# EUROPEAN PATENT APPLICATION

(11) **EP 3 494 925 A1**
(43) Date of publication of application: **12.06.2019**
(21) Application number: 18210838.1
(22) Date of filing: 06.12.2018
(51) Int. Cl.: A61F 2/07, A61F 2/844, A61F 2/06, A61F 2/82

(54) **STENT GRAFT**

(30) Priority: 07.12.2017 US 201762595671 P
(71) Applicant: Cook Medical Technologies LLC, Bloomington, IN 47404 (US)
(72) Inventor: KIM, Woong, West Lafayette, IN 47906 (US); KRATZBERG, Jarin, West Lafayette, IN 47906 (US); SVENDSEN, Mark, Bloomington, IN 47401 (US); ROEDER, Blayne A., Bloomington, IN 47401 (US)
(74) Representative: Williams Powell

(57) **Abstract**

A stent graft device (10) comprises a main tubular portion (21) and a side branch (31) in fluid communication with the main tubular portion, the side branch comprising a ringed structure (30) comprising a first ring (50) having four contact points (52-58) spaced approximately equally about the circumference thereof, wherein in an expanded configuration (18), a first contact point and third contact point define a diameter of the first ring therebetween, and a second contact point and fourth contact point define a diameter of the first ring therebetween, and wherein in a collapsed configuration the first contact point is disposed adjacent the third contact point, and the second contact point is disposed adjacent the fourth contact point. The device may include a plurality of radiopaque rings and associated structures, which are folded in a predictable fashion prior to crimping. A mandrel may be employed for biasing the end rings of a ringed structure. A biodegradable adhesive may be used to assist in folding.

## Description

### BACKGROUND

The present application generally relates to medical devices, in particular stent grafts. More particularly, the present application relates to a stent graft and a method of making the stent graft involving a compact architecture for loading and delivery of the stent graft, especially by high packing density pre-folding of side branch end rings.

Treatment of aneurysms has included the use of stent grafts that are implanted within the vascular networks and that include one or more stents affixed to graft material. The stent grafts may be secured at a treatment site by endovascular insertion utilizing introducers and catheters, after which they are enlarged radially and remain in place by attachment to the vessel wall. In particular, stent grafts are known for use in treating descending thoracic and abdominal aortic aneurysms where the stent graft at one end defines a single lumen for placement within the aorta and at the other end is branched to form a plurality of lumens, for extending into the branch arteries.

During implantation, which is typically conducted through the vasculature of the patient rather than by open surgery, the progress of the procedure may be monitored by a practitioner using a visualization method, such as fluoroscopy. Visualization methods increase the success of an implantation procedure as they ensure that the correct portions of the device are aligned with, or placed in, the appropriate anatomical features to be treated. However, because the stent graft is introduced in a crimped or compressed format, there may be ambiguity as to which components are truly being viewed by the practitioner.

It has been a challenge to develop a stent graft architecture which has a predictable crimped or collapsed configuration which can be easily recognized and comprehended when viewed by a visualization method, such as fluoroscopy.

### SUMMARY

In one aspect, the present disclosure provides a stent graft for placement in a body vessel of a patient. The stent graft has an expanded configuration and a collapsed configuration. The stent graft may include a main tubular portion having a sidewall and defining a main lumen. The stent graft may include at least one aperture being formed through the sidewall. The stent graft may include at least one side branch which may be a tubular body extending from a first end to a second end. At least one of the side branches may include a graft material and defines a side lumen therethrough. The side branch may be attached to the main tubular portion at an aperture formed in the main branch such that each side lumen is in fluid communication with the main lumen. The side branch may include a ringed structure enclosed within the graft material. The ringed structure may include a first ring disposed along a length of the side branch, and may include a second ring; and may include at least one rib extending from the first ring to the second ring. The first ring includes four contact points spaced approximately equally about the circumference thereof. In the expanded configuration, a first contact point and third contact point define a diameter of the first ring therebetween, and a second contact point and fourth contact point define a diameter of the first ring therebetween. In the collapsed configuration, the first contact point is disposed adjacent the third contact point, and the second contact point is disposed adjacent the fourth contact point.

In another aspect, the present disclosure provides a method of making a stent graft having an expanded configuration and a collapsed configuration. The method includes folding a first end of a tubular body to form a folded end, the tubular body extending from the first end to a second end, the tubular body comprising a graft material and defining a lumen therethrough and including a ringed structure enclosed within the graft material. The ringed structure may includes a first ring disposed along a length of the side branch, and may include a second ring disposed along a length of the side branch, and may include at least one rib extending from the first ring to the second ring. The first ring includes four contact points spaced equally about the circumference thereof. In the expanded configuration, a first contact point and third contact point define a diameter of the first ring therebetween, and a second contact point and fourth contact point define a diameter of the first ring therebetween. Folding the first end may include a step of inserting a superelastic wire in the lumen and through the graft material proximate the first contact point. The method may include a step of threading the superelastic wire through the graft material proximate the third contact point. The method may include a step of pulling the superelastic wire such that the first contact point is disposed adjacent the third contact point. The method may include a step of threading the superelastic wire through the graft material proximate the second contact point. The method may include a step of threading the superelastic wire through the graft material proximate the fourth contact point. The method may include a step of pulling the superelastic wire such that the second contact point is disposed adjacent the fourth contact point to yield the folded end.

In another aspect, the present disclosure provides a method of making a stent graft having an expanded configuration and a collapsed configuration. The method includes folding a first end of a tubular body to form a folded end, the tubular body extending from the first end to a second end, the tubular body comprising a graft material and defining a lumen therethrough and including a ringed structure enclosed within the graft material. The ringed structure may includes a first ring disposed along a length of the side branch, and may include a second ring disposed along a length of the side branch, and may include at least one rib extending from the first ring to the second ring. The first ring includes four contact points spaced equally about the circumference thereof. In the expanded configuration, a first contact point and third contact point define a diameter of the first ring therebetween, and a second contact point and fourth contact point define a diameter of the first ring therebetween. Folding the first end may include a step of moving the first contact point such that it is disposed adjacent the third contact point, and applying an adhesive to secure the first contact point to the third contact point. The method may include a step of bringing the second contact point adjacent the fourth contact point to yield the folded end.

Further objects, features and advantages of this system will become readily apparent to persons skilled in the art after a review of the following description, with reference to the drawings and claims that are appended to and form a part of this specification.

### BRIEF DESCRIPTION OF THE DRAWINGS

FIG. 1 is a schematic side view of a stent graft constructed in accordance with the principles of the present invention;
FIG. 2A is a side view of a stent graft and folding method resulting in a less-orderly folding scheme;
FIG. 2B is a view of the end rings of the device of FIG. 2A when in a crimped condition;
FIG. 3A is a schematic end view of an end ring of a side branch of a stent graft when being folded in accordance with an aspect of the present disclosure;
FIG. 3B is a perspective view of an end ring of a side branch of a stent graft when being folded in accordance with an aspect of the present disclosure;
FIG. 4A is a of a stent graft and folding method resulting in a less-orderly folding scheme;
FIG. 4B is a radiograph displaying a side view of a stent graft folded in accordance with an embodiment of the present disclosure;
FIG. 5 is an illustration of the steps of a method for prefolding a side branch of a stent graft in accordance with an embodiment of the present disclosure;
FIG. 6A is a perspective view of a ringed structure being set over a mandrel in accordance with an embodiment of the present disclosure;
FIG. 6B is an end view of the ringed structure and mandrel of FIG. 6A;
FIG. 7 is a view of the ringed structure as disclosed in FIG. 6;
FIG. 8 is a series of side views of method steps for folding an end of a side branch in accordance with an embodiment of the present disclosure;
FIGs. 9A-9C are views of a side branch having a ringed structure as in FIG. 7 in an open configuration;
FIGs. 9D-9E are views of the side branch of FIGs. 9A-9C in a closed configuration; and
FIG. 10 is a view of a partially-folded end of a side branch the folding of which is aided by an adhesive, in accordance with another embodiment of the present disclosure.

### DETAILED DESCRIPTION

The drawings are purely schematic illustrations of various aspects of the invention and are not necessarily to scale, unless expressly stated.

The terms "substantially" or "about" used herein with reference to a quantity includes variations in the recited quantity that are equivalent to the quantity recited, such as an amount that is equivalent to the quantity recited for an intended purpose or function. "Substantially" or derivatives thereof will be understood to mean significantly or in large part. When used in the context of a numerical value or range set forth, "about" or "substantially" means a variation of ±15%, or less, of the numerical value. For example, a value differing by ±15%, ±14%, ±10%, or ±5%, among others, would satisfy the definition of "about."

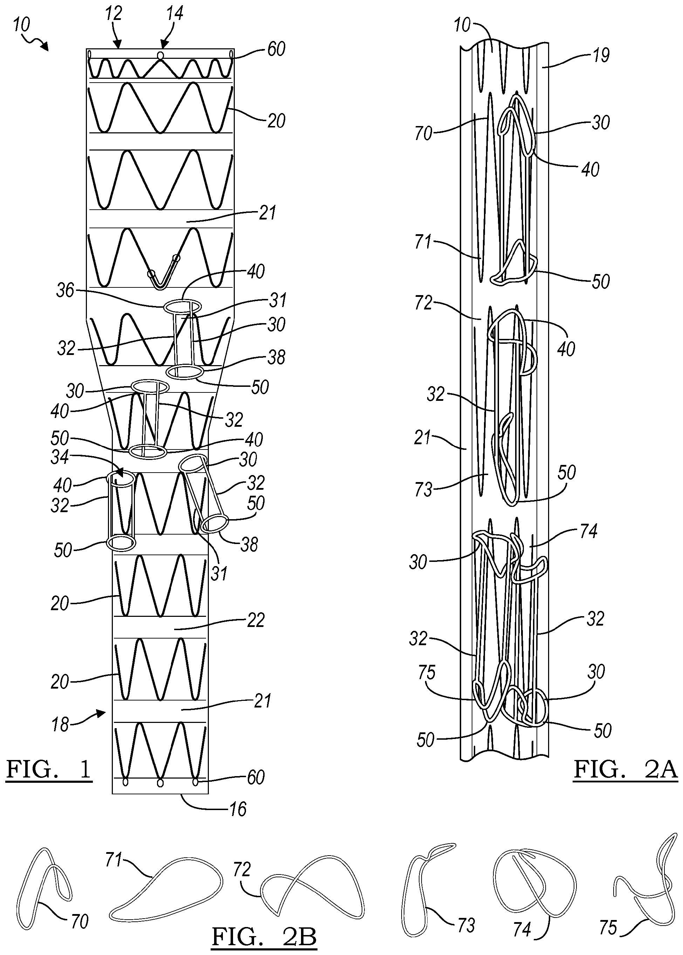

In the first embodiment, a stent graft 10 is illustrated. The stent graft 10 extends from a first end 14 to a second end 16 and defines a lumen 12 therethrough. As shown in Figure 1, the stent graft 10 is shown in an expanded configuration 18. The stent graft 10 may be made up of a series of stent rings 20, which, on the main branch 21, are concentric and disposed at longitudinal intervals along the length of stent graft 10. The stent rings 20 surround and support at least one piece of graft material 22, which may be in sheet or tubular form, and which forms an inner surface of the stent graft 10 and defines a lumen 12 running therethrough. The stent graft 10 may optionally include at least one radiopaque marker 60, which in some cases may be located at an end of the stent graft 10, such as at first end 14, or at second end 16, or at both ends.

The stent rings 20 may be self-expanding or balloon-expandable elements. As illustrated, the stent rings 20 are defined by a plurality of struts arranged in a zigzag shape. In one embodiment, the rings 20, and struts of the stent ring, can be laser cut from a metallic tube or cannula. The metal may be suitably heat treated to make it deformable and resilient. Alternatively, the rings 20 may be made from both a shape memory alloy (such as a nickel-titanium alloy) and the rib or connector 32 made from stainless steel wire, these two components being joined by crimping with a small tube.

The stent rings 20 may be manufactured in diameters of from about 3 millimeters (mm) to about 100 mm, or from about 3 mm to about 40 mm, and lengths of from about 3 mm to about 40 mm. The wire from which the stent ring may be formed may have a diameter or thickness of from approximately 35 microns to approximately 1000 microns, or about 35 microns to about 500 microns. Where the stent is formed by laser cutting from a metal tube, the diameter of the metal tube may have a diameter of from 3 mm to 40 mm and have a wall thickness of from about 35 microns to about 1000 microns. Each stent ring may be formed from a single piece of wire.

The graft material 22 may be a tubular graft material, and may be nonporous so that it does not leak or sweat under physiologic forces. The graft material may be made of a biocompatible material, including but not limited to a DACRON® polyester, another polyester fabric, polytetrafluoroethylene (PTFE), expanded PTFE, THORALON®, a polyamide, and other synthetic materials known to those of skill in the art. Naturally occurring biomaterials, such as collagen, particularly a derived collagen material known as extracellular matrix (ECM), such as small intestinal submucosa (SIS), may also be employed. In some embodiments, the graft material 22 may be constructed as a preshaped tube. In some embodiments, the graft material 22 may be a woven material.

As illustrated in Figure 1, in one embodiment the stent graft 10 may include a number of side branches 31 extending from the main body of the stent graft 10. Multi-branched stent grafts are known in the field of thoracoabdominal aortic artery (TAAA) repair, with the smaller side branches 31 being used for placement adjacent or in smaller branching vessels running from the aorta. Each side branch 31 may be connected to the main branch 21 at a fenestration formed through the graft material of the main branch 21, and may extend to a free end 38, defining a side branch lumen 34 therebetween and in fluid communication with the lumen of the main branch. Each side branch 31 may be connected at a different point along its length to the main branch 21. In some embodiments, the side branch 31 may be connected to the main branch 21 by a connected end 36. In other embodiments, the side branch may be connected to the main branch 21 at a different point along its length between the ends.

In the embodiment illustrated, the side branch 31 is supported by a ringed structure 30. Each ringed structure includes a first ring 50, and may include a second ring 40. In some embodiments, the ringed structure 30 may also include a connector 32 which is attached to and joins first ring 50 to second ring 40, and provides axial spacing therebetween.

In some embodiments, the ringed structure 30 may be rendered radiopaque in order to provide for better visualization during delivery and implantation of the stent graft 10. In one embodiment, this may be achieved by wrapping the ringed structure in a radiopaque material, such as a platinum wire. In other embodiments, the ringed structure 30 may be provided with a radiopaque coating, or a material other than platinum, including but not limited to gold, palladium, tungsten, and tantalum, or a mixture of any of these materials, may be employed.

The stent graft 10 of Figure 1 is illustrated in a compressed or crimped condition 19 in Figure 2A. In this figure, the crimping or radial compression has been achieved using a method of crimping which lacks the pre-folding step. As can be seen in Figure 2A, the first rings 50 and second rings 40 of the ringed structures 30 take on a variety of haphazard shapes after crimping, as exemplified by misfolded rings 70, 71, 72, 73, 74, and 75. The shapes of misfolded rings 70-75 are shown independent of the structure of the device in Figure 2B for increased clarity. When visualized during a procedure, the misfolded rings 70-75 may be difficult or impossible to deconvolute, leading to potential errors in placement of the implant.

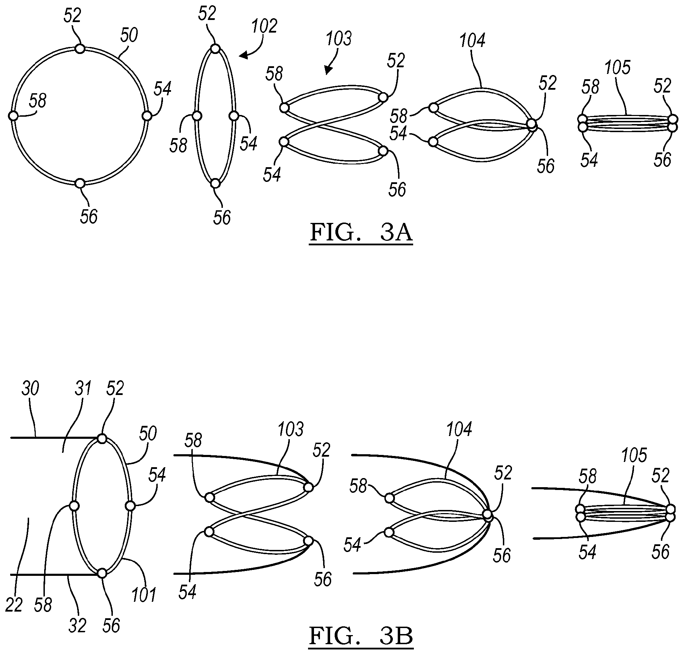

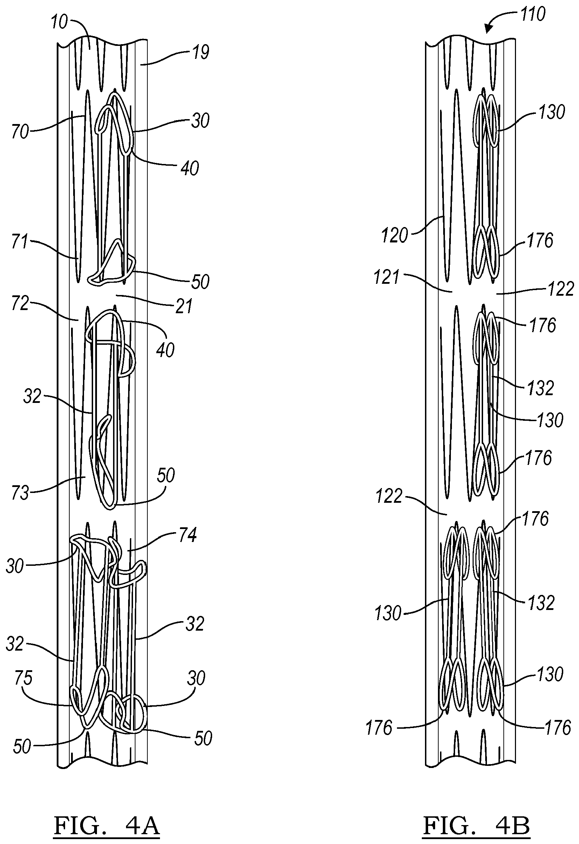

Figure 3A illustrates a method of pre-folding an end ring of a side branch, or of a stent graft, in accordance with an embodiment of the present disclosure. Pre-folding can yield well-defined folded structures that are readily visible and distinguishable during visualization procedures (as can be seen in Figure 4B, contrasted with the difficult-to-discern structures of Figure 4A from the method lacking pre-folding.) In the embodiment of Figure 3A, the first ring 50 defines four different contact points 52/54/56/58 about its circumference.

In one embodiment, these four contact points may simply be geographical markers, which may not define additional components of the ring 50. In other embodiments, the ring 50 may be constructed such that some or all of the contact points 52/54/56/58 have properties differing from the remainder of the ring 50 such that folding is facilitated by these differences. In one embodiment, the ring 50 may be bent at some or all of the contact points 52/54/56/58. In another embodiment, some or all of the contact points 52/54/56/58 may be constructed to be thinner than the thickness of the remainder of the ring 50, thereby predisposing the thinner contact points to bending first when the ring 50 is compressed.

First contact point 52 is disposed approximately 90 degrees counterclockwise of second contact point 54, which is likewise disposed about 90 degrees counterclockwise of third contact point 56, which is likewise disposed about 90 degrees counterclockwise of fourth contact point 58. Put another way, the four contact points 52, 54, 56, and 58 may be considered substantially equivalent to the 12:00, 3:00, 6:00, and 9:00 positions of a standard clock face.

A folding scheme for a ring 50 of such construction is now described, and depicted in Figure 3A and 3B. The end ring 50 starts in first configuration 101. In first folding step, the ring is moved to configuration 102, wherein the ring 50 is folded such that second contact point 54 is brought into proximity with fourth contact point 58. In folding step 103, the ring 50 is then compressed such that the first contact point 52 is brought into proximity of third contact point 56. In the next step, the ring is folded into configuration 103, in which the ring 50 takes on a U-shape, or a C-shape. In configuration 104, the first contact point 52 is brought adjacent third contact point 56, and in configuration 105, the second contact point 54 is brought adjacent the fourth contact point 58. After the final step, the compressed configuration of the end ring 50 is defined. This configuration 105 can be easily distinguished from other components of the device 10 under visualization during an implantation procedure. The configurations of the ring 101, 102, 103, 104, and 105 are shown in the context of the side branch 31 of a stent graft 10 in Figure 3B, which demonstrates how the free end 38 compresses after such a folding regimen.

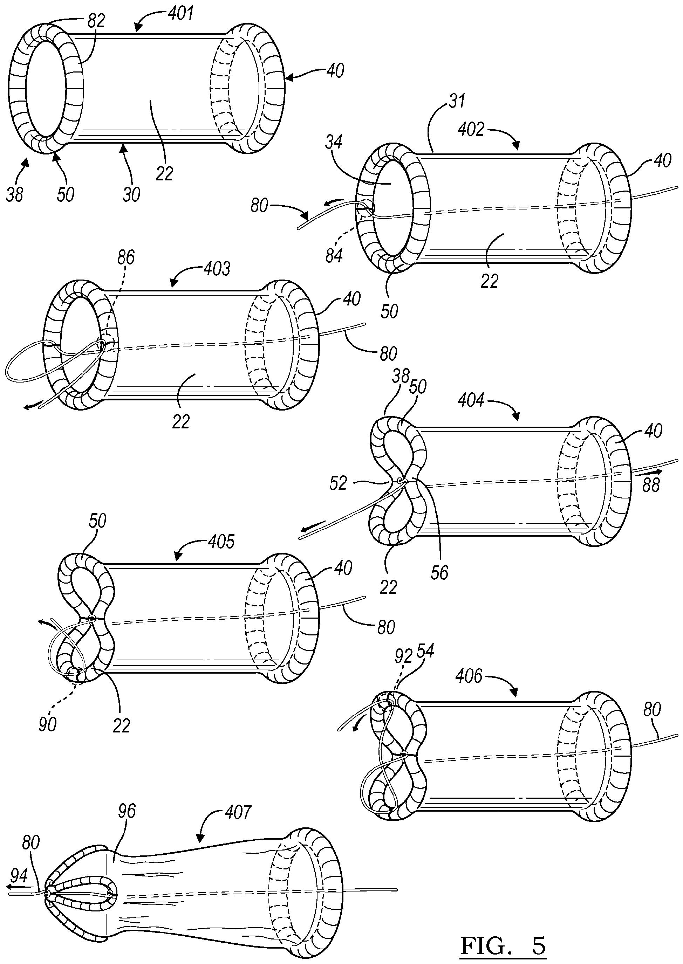

Figure 5 illustrates one particular method of achieving the folding of an end of a stent graft, or the end of a side branch of a stent graft. The side branch 31 is shown in its expanded configuration 18 in step 401 of Figure 5. The ringed structure 30 is encompassed by graft material 22, and so is not directly visible in the illustration. The graft material surrounds first ring 50 and second ring 40, and may be attached to the ring structures buy a plurality of blanket stitches 82.

In step 402, a superelastic wire 80 is run through the lumen 34 of the side branch 31 and threaded through the graft material 22 which lies proximate and surrounding the first ring 50 at first contact point 52. In one embodiment, the superelastic wire 80 may be made of a shape memory alloy, such as a nickel titanium alloy. Additionally, the superelastic wire 80 may be coated with a low-friction material, such as polytetrafluoroethylene (PTFE) in order to facilitate easy threading and removal of the wire.

In step 403, the superelastic thread 80 is threaded through a portion 86 of the graft material 22 which is proximate third contact point 56 of the end ring 50. In step 404, the superelastic wire is pulled taut, optionally in both the directions of first end 36, and free end 38, to bring first contact point 52 and second contact point 56 into proximity of one another. In step 405, the superelastic wire 80 is threaded through a portion 90 of the graft material 22 which is proximate fourth contact point 58. In step 406, the superelastic thread is then threaded through the graft material 22 at a position 92 proximate second contact point 54. In the finally illustrated step 407, the superelastic wire 80 may be pulled in direction 94 and may be pulled free of the side branch 31 in order to yield closed end 96.

As shown in Figure 4B, the use of such a folding scheme results in a stent graft 110 that has a compressed configuration 119 that gives rise to predictably folded end rings 176 of ringed structures 130. This is in contrast to the structure seen in Figure 4A, which is the result of crimping without pre-folding.

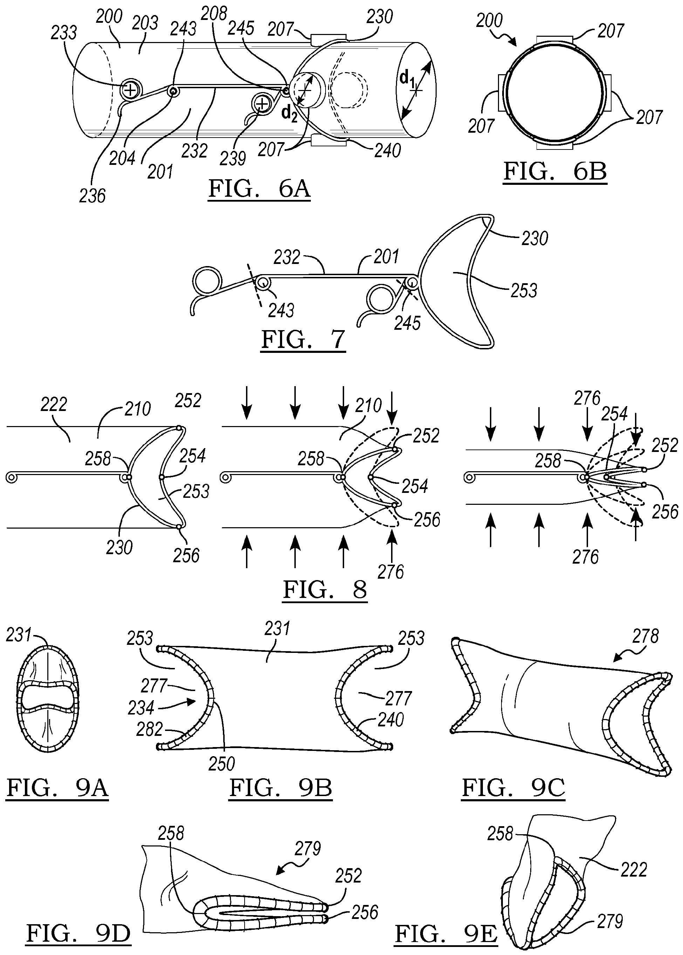

In order to prepare the end rings to be folded, the ringed structure may be made by setting over a mandrel, as shown in Figure 6A, in order to bias it to a partially-folded state.

As shown in Figure 6A, the mandrel 200 includes four rounded protrusions 237 on its outer surface 235. These protrusions 237 can be formed of separate elements attached to the cylindrical body of the mandrel, such as screws, or can be formed from the outer surface 235 of the mandrel 200 itself. The ringed structure 230 is formed by placement of a wire 236 over the outer surface 235 of the mandrel, and winding the wire to form end ring 240. The mandrel 200 has a first diameter D1, which may define the diameter of the side branch or of the stent graft in which the ringed structure 230 is to be employed. Each protrusion 237 has a diameter D2 in order to define a radius of curvature for the partially collapsed form of the end ring 240.

The mandrel made further and optionally include locking screws, such as screw 233 and screw 239, in order to keep the wire 236 in place on the outer surface 235 of the mandrel 200. Further anchoring elements such as anchor 244 and anchor 208 as shown in Figure 6A may also be employed to further stabilize the wire 236.

In one embodiment, the ringed structure 230 may be shaped by heat setting the wire 236 over the mandrel 200. The wire 236, which may be made of a shape memory material, such as nickel titanium alloy, can be pre-coiled with a radiopaque wire thereabout, such as a wire made of platinum, prior to engagement with the mandrel 200. In one embodiment, the wire 236 may be heat set, for example at about 400 degrees Celsius for about five minutes, and then cooled, such as by quenching in water. In one embodiment, when the wire 236 is shaped into the ringed structure 230, it may include two rings 250 and 240, on opposing sides of, and connected to, a connector 232.

As shown in Figure 7, the finished ringed structure 230 including rib or connector 232 may attain its final shape by cutting of excess wire at the positions indicated by dashed lines. As shown in Figure 7, the final structure includes a fish mouth shape 253 of ring 230. As can further be seen in Figure 7, the wire 201 and ring structure 230 may be formed to include a pair of hoops 243 and 245, which are formed by winding the wire 236 around anchors 233 and 239 of mandrel 200. These hoops 243/245 may be used as a junction point to assist proper fixation to a graft material during blanket suture sewing when the graft material is disposed over the ringed structure 230.

Figure 8 illustrates a compression scheme for a stent graft or a side branch 210 including a ringed structure 230 which has been preset to a fishmouth shape 253. The fishmouth shape assists in bringing first contact point 252 into proximity with third contact point 256 as a crimping force 276 is applied to the side branch 210. The crimping force may be a substantially radially-directed force, which also brings second contact point 254 into proximity of fourth contact point 258.

Figures 9A-9E illustrate a side branch or a stent graft in accordance with the embodiments described herein. As can be best seen in figures 9B and 9C, both the first ring 240 and the second ring 250 of the ringed structure 230 are shown in a fishmouth configuration 253. Blanket stitching 282 holds the graft material 231 on to the ring structure 230. Each fishmouth in figures 9B and 9C are illustrated in an open configuration 277. Side and perspective views, respectively, are displayed in Figures 9D and 9E. As shown, the end of the side branch 210 is instead in a closed position 279. As can be seen in Figure 9D, first contact point 252 has been brought into proximity with third contact point 256. Second contact point 254 is also in proximity of and adjacent to fourth contact point 258.

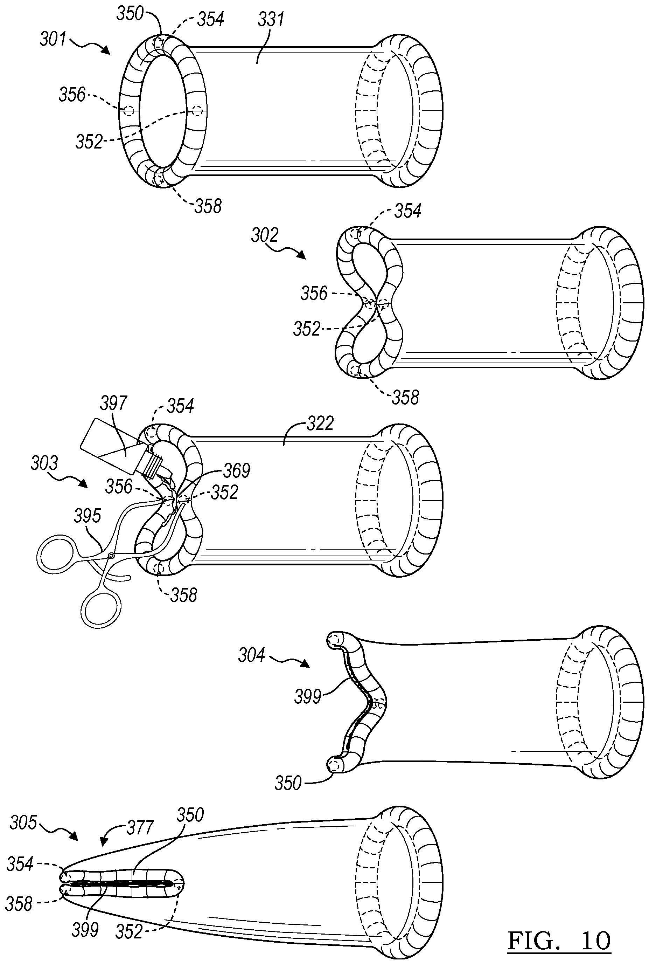

In another embodiment, and as illustrated in figure 10, the stent graft 331 according to the principles of the present disclosure may be constructed by a method that strategically employs an adhesive to aid in achieving the collapsed or closed configuration. The unfolded side branch 331 (or stent graft) is illustrated in a first step 301. In second step 302, the first contact point 352 has been folded and is in proximity of third contact point 356.

In step 303, an adhesive 397 may be applied to junction 369, which is formed by the contact between the graft material 322 covering first contact point 352 and third contact point 356. Optionally, a crimping tool 395 or a clamp may be used to hold the first contact point 352 to third contact point 356 in order to allow the adhesive to set at the junction 369.

The adhesive 397 may be a biocompatible, and in some instances a bioresorbable material. Because the adhesive will be exposed to the bloodstream of the patient in whom the device will be deployed, the adhesive should break down into digestible components that may safely circulate in the vascular system. In one example the adhesive may include a molten sugar, such as glucose. Sugar constantly circulates in the bloodstream as an energy source for the body, so its introduction on an implant such as a stent graft will not provoke a negative response in the patient. In one embodiment, the sugar is applied as a molten sugar, which is viscous and adheres readily to many materials. Additionally, molten sugar will harden as it cools, and the crimping procedure will pulverize the hardened sugar on the device. This solidified, pulverized sugar allows for easy dissolution in the bloodstream during and after the deployment procedure. Other examples biocompatible and/or bioresorbable adhesives include, but are not limited to, polymers including low molecular weight polyethylene glycol, fibrin glue, hydrogels including those generated by mixing aldehyded dextran and s-poly(L-lysine), and other carbohydrates.

In step 304, the adhesive has set to form interface 399. Finally, in step 305, second contact point 354 can be brought into proximity with and adjacent to fourth contact point 358 in order to achieve closed structure 377 of ring 350. Adhesive 399 can be seen at the interface at which the folded halves of the ring 350 meet.

As a person skilled in the art will readily appreciate, the above description is only meant as an illustration of implementation of the principles this application. This description is not intended to limit the scope of this application in that the system is susceptible to modification, variation and change, without departing from the scope of this application, as defined in the following claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/595,671, from which this application claims priority, and in the abstract accompanying this application are incorporated herein by reference.

## Claims

1. A stent graft for placement in a body vessel of a patient, the stent graft having an expanded configuration and a collapsed configuration, the stent graft comprising:
a main tubular portion comprising a sidewall and defining a main lumen, at least one aperture being formed through the sidewall, and
at least one side branch comprising a tubular body extending from a first end to a second end, the at least one side branch comprising a graft material and defining a side lumen therethrough, the at least one side branch being attached to the main tubular portion at an aperture formed in the main tubular portion such that the side lumen is in fluid communication with the main lumen, the at least one side branch comprising a ringed structure enclosed within the graft material, the ringed structure comprising a first ring having a circumference, the first ring disposed along a portion of the side branch;
wherein the first ring comprises four contact points spaced approximately equally about the circumference thereof,
wherein in the expanded configuration, a first contact point and third contact point define a diameter of the first ring therebetween, and a second contact point and fourth contact point define a diameter of the first ring therebetween, and
wherein in the collapsed configuration, the first contact point is disposed adjacent the third contact point, and the second contact point is disposed adjacent the fourth contact point.

2. The stent graft of claim 1, wherein the ringed structure comprises a shape memory alloy.

3. The stent graft of claim 1 or 2, wherein a portion of the ringed structure is surrounded by a radiopaque material.

4. The stent graft of claim 3, wherein the first ring is surrounded by a radiopaque material.

5. The stent graft of any preceding claim, wherein the ringed structure further comprises a second ring, and a rib extending from the first ring to the second ring.

6. The stent graft of claim 5, wherein the first ring is disposed at the first end of the side branch, and the second ring is disposed at the second end.

7. The stent graft of claim 5 or 6, wherein at least one of the first ring and the second ring is surrounded by a radiopaque material.

8. The stent graft of any preceding claim, wherein at least one of the four contact points comprises a bent portion of the first ring.

9. The stent graft of any preceding claim, wherein at least one of the four contact points has a first thickness less than a second thickness of a portion of the first ring.

10. A stent graft for placement in a body vessel of a patient, the stent graft having an expanded configuration and a collapsed configuration, the stent graft comprising:
at least one side branch comprising a tubular body extending from a first end to a second end, the at least one side branch comprising a graft material and defining a side lumen therethrough, the at least one side branch comprising a ringed structure enclosed within the graft material, the ringed structure comprising a first ring having a circumference, the first ring disposed along a portion of the side branch;
wherein the first ring comprises four contact points spaced approximately equally about the circumference thereof,
wherein in the expanded configuration, a first contact point and third contact point define a diameter of the first ring therebetween, and a second contact point and fourth contact point define a diameter of the first ring therebetween, and
wherein in the collapsed configuration, the first contact point is disposed adjacent the third contact point, and the second contact point is disposed adjacent the fourth contact point.

11. The stent graft of claim 10, wherein the ringed structure comprises a shape memory alloy.

12. The stent graft of claim 10 or 11, wherein a portion of the ringed structure is surrounded by a radiopaque material.

13. The stent graft of any of claims 10 to 12, wherein the ringed structure further comprises a second ring, and a rib extending from the first ring to the second ring.

14. The stent graft of claim 13, wherein the first ring is disposed at the first end of the side branch, and the second ring is disposed at the second end.

15. The stent graft of any of claims 10 to 14, wherein at least one of the four contact points has a first thickness less than a second thickness of a portion of the first ring.
